(19) Europäisches Patentamt
European Patent Office
Office européen des brevets

(11) Publication number: **0 249 262**
**A2**

(12) # EUROPEAN PATENT APPLICATION

(21) Application number: **87200903.0**

(51) Int. Cl.4: **C08G 59/00**, C08G 59/02

(22) Date of filing: **14.05.87**

(30) Priority: **09.06.86 US 871951**
**09.06.86 US 871952**

(43) Date of publication of application:
**16.12.87 Bulletin 87/51**

(84) Designated Contracting States:
**DE FR GB IT NL SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Dewhirst, Kenneth Carl**
**410 Bayou Cove**
**Houston Texas 77042(US)**

(74) Representative: **Aalbers, Onno et al**
**P.O. Box 302**
**NL-2501 CH The Hague(NL)**

(54) **Thermoplastic polymer composition having thermosetting processing characteristics.**

(57) The present invention relates to a new polymer composition having the processing characteristics of a thermosetting polymer along with an improved balance of properties including improved modulus/glass transition temperature/toughness balance. These new polymer compositions are prepared by reacting certain diphenolic compounds with certain diepoxide compounds to form linear units which are lightly crosslinked through the resulting secondary hydroxyl groups. Also disclosed and claimed are processes for preparing such compositions, cured compositions and end-use applications.

EP 0 249 262 A2

Xerox Copy Centre

# THERMOPLASTIC POLYMER COMPOSITION HAVING THERMOSETTING PROCESSING CHARACTERISTICS

The present invention relates to novel thermoplastic polymer compositions which may be converted into thermoset composite structures by typical thermosetting resin methods. In particular, the present invention relates to thermoplastic compositions having flexible portions and bulky stiff portions in their molecular structures, prepared by reacting a polyepoxide with a polyphenol to form linear molecules and crosslinking to a certain degree the resulting molecules.

Epoxy compositions and their curing techniques are well known, and the patents issued on curable epoxy compositions number in the thousands. It will be appreciated that each and every one of the known epoxy-curing systems has its pros and cons and that there remains a continuing need to develop better epoxy compositions.

There is, in particular, an increasing need in the aerospace and automotive sectors for high performance thermosetting compositions or matrices for fibre reinforced composites. Fibre reinforced composites are very desirable in aerospace applications because they can offer a combination of good stiffness, strength and are light weight. Increasingly, the aerospace manufactures have demanded higher performance from the thermoset resins used in fibre reinforced composites. These higher performance thermoset resins are expected to possess these following characteristics:

-good mechanical properties at temperatures above about 90 °C
-good thermal oxidative stability
-good toughness properties, including good impact resistance
-good fatigue properties
-good chemical and solvent resistance
-good fire resistance
-high resistance to humidity, e.g., the "hot-wet" properties of the composite must remain high.

A further, and very important property of such systems is that the composite must have acceptable processing characteristics. For example, the current techniques for manufacturing aerospace components typically involves the use of prepregs and laminates, which are cured by applying heat and pressure in a vacuum bag/autoclave-type apparatus. Therefore, the most desirable thermosetting resin compositions should be processable on the standard equipment currently utilized in the aerospace industry.

A broad spectrum of thermosetting epoxy resin systems is currently being used by the aerospace industry, primarily as composite matrices and adhesives. As a class, epoxy resin systems are very versatile materials offering, as mentioned above, chemical resistance, high adhesive strength, good electric properties and are easy to use or process into composites. However, to improve their high temperature properties, such current epoxy resin systems must be highly crosslinked. This crosslinking, however, results in generally lower toughness.

There are currently various engineering thermoplastics (not thermosetting polymers) that offer excellent high temperature properties along with high toughness. One such example currently being investigated is the use of poly(ether-ether)ketone ("PEEK") as the matrix. However, there are processing problems with the use of PEEK and other similar engineering thermoplastic resins since such materials not only are difficult to process on thermoplastic apparatus (i.e., difficult to extrude), but also do not lend themselves to processing by the thermosetting techniques now currently in use by the aerospace industry.

What is needed is a resin system that not only combines the good property advantages of such high performance engineering thermoplastics such as PEEK, but is also processable as a thermosetting resin matrix.

Thermoplastic polyethers having relatively high impact strength are disclosed and claimed in US-A 3,637,590, while the process for preparing such polymers is claimed in US-A 3,306,872. Even though such polymers, which are based on the reaction product of certain diepoxides with certain bisphenols, have improved impact strength, such polymers lack adequate high temperature properties and solvent resistance for high performance applications. Similar compositions having improved impact strength are also disclosed in DE-A 2,142,579 where certain diepoxides are reacted with certain diphenols (e.g. 2,2-bis(4-hydroxynaphth-1-yl)propane) to produce polymers for eyeglasses.

Other epoxy systems such as those disclosed in US-A 4,473,674, are touted for aerospace applications. The composites described in this reference are based on multifunctional epoxies, such as tetraglycidylmethylenedianiline. Such systems also have important deficiencies as discussed in the various examples which follow.

The present invention aims at achieving a thermoplastic composition having the processing characteristics of a thermosetting polymer along with an improved balance of properties including solvent resistance and improved modulus/glass transition temperature/toughness balance. Thereto the invention relates to a composition comprising linear molecules having the repeating structures:

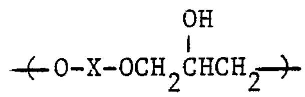 and I

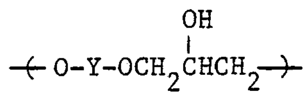 where: II

a) "X" represents a stiff segment comprising stiff units (SU) and optional flexible units (FU), which stiff units and flexible units are interconnected;

b) "Y" represents a flexible segment comprising stiff units (SU') and optional flexible units (FU') which stiff units and flexible units are interconnected;

c) said stiff units, SU and SU', are independently selected from the group consisting of substituted and non-substituted aromatic rings, cycloaliphatic rings and non-interfering heterocyclic rings;

d) said flexible units, FU and FU', are independently selected from the group consisting of

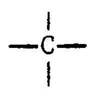, 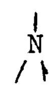, 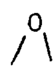, 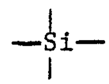, 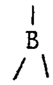 and 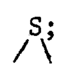;

e) the number of stiff segments in said molecules is "a", the number of flexible segments in said molecules is "b",

and the ratio $\frac{a}{a+b}$ is less than

or equal to one; and

f) the ratio of the number of stiff units to flexible units in said stiff segment (SU/FU) is equal to or greater than the ratio of the number of stiff units to flexible units in said flexible segment (SU'/FU').

As shown in the examples which follow, Applicants have discovered a new method for preparing novel polymers wherein it is now possible to obtain both high temperature performance and high toughness, i.e., Applicants have discovered a means to uncouple the usual temperature/toughness balance relationship. In particular, in a preferred embodiment Applicants have prepared polymers having the following property set:
Glass transition temperature, Tg = 175 °C (DSC)
Fracture toughness, $K_q$ = 2.5 KSI $^{0.5}$ in. (Compact Tension)

$$\text{Flex modulus, } E = \begin{cases} 390 \text{ KSI (Dry R.T.)} \\ 350 \text{ KSI (Wet 200 °F)} \end{cases}$$

Water gain, $\Delta W/W_o$ = 1.45% (saturation).

In other examples, Applicants have shown that particular polymers, having particular ranges of stiff segments and flexible segments, possess truly extraordinary properties, especially relating to solvent resistance.

There are two basic aspects to the present invention: one involves the process for making certain thermoplastic polymers and the other involves the polymers as compositions-of-matter.

I. Process

In a broad sense, the present invention relates to a mixture of a diphenoxy component and a diepoxide component for making a prepolymer composition, which may be reacted with a condensation catalyst.

A. Diphenoxy Component

In a preferred embodiment the diphenoxy components employed herein have the structure HO-X-OH or HO-Y-OH where "X" represents the stiff segment specified above and "Y" represents the flexible segment specified above.

As a practical matter, it is preferred that the diphenoxy component contain the stiff segment, i.e. that the phenoxy component be HO-X-OH. The reason for this is that it is easier to synthesize the diphenoxy component (containing the relatively large number of stiff units) than it is to glycidate the corresponding diphenoxy compound. In particular, it is preferred to employ diepoxides based on BPA and use diphenoxy compounds based on the less common compounds. However, in certain cases it may be preferable to have the stiff segment, X, in the diepoxide component since the diepoxide may have a lower melting point than the diphenoxy component, resulting in an easier thermoplastic polymer synthesis, especially if it is desired to perform the synthesis in the melt as opposed to a solution preparation.

Preferably it is desired that the diphenoxy compound be meta or para derivatives as opposed to an ortho structure.

An important aspect of the present invention is the selection of stiff units and flexible units such that the resulting polymer molecules have the appropriate type and ratio of stiff units to flexible units.

By use of the term "flexible units " are meant those units that permit rotation at an angle. Examples of such flexible units are

Broad group Examples

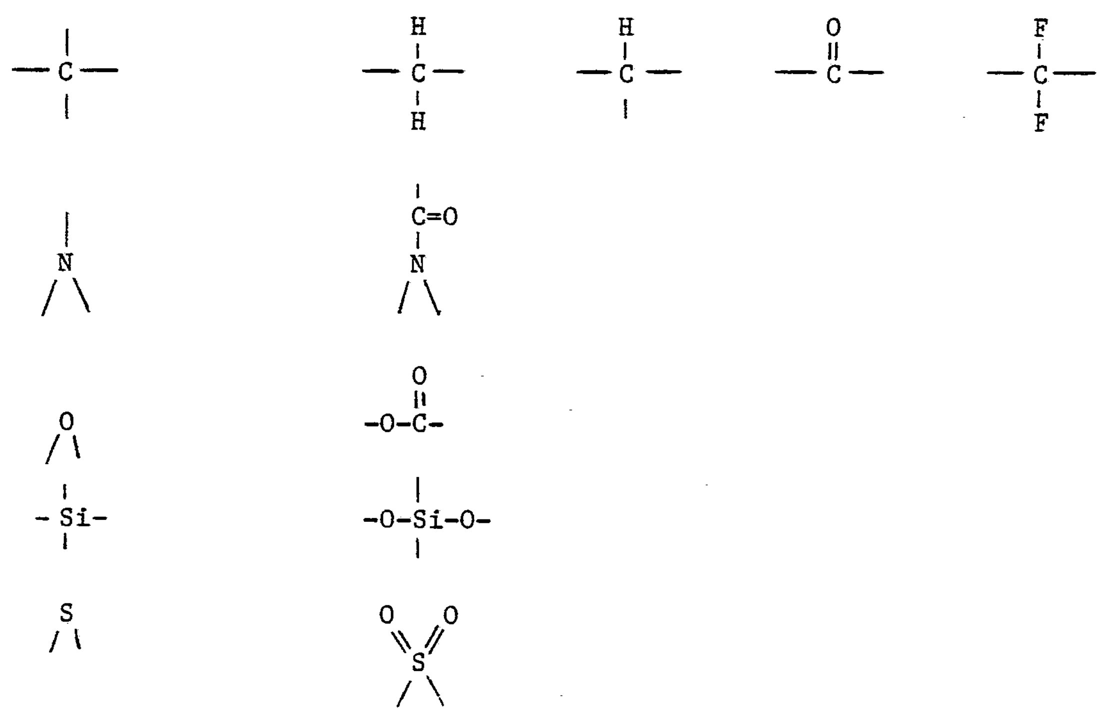

The stiff units are selected from the group consisting of substituted or non-substituted aromatic rings, cycloaliphatic rings and heterocyclic rings. The aromatic rings are inertly substituted or un-substituted benzene radicals. Substituted benzene radicals have substituents which do not interfere in the process independently selected from the group consisting of Cl, Br or $C_1$-$C_5$ alkyl groups. Annulation of benzene rings gives rise to

naphthalene 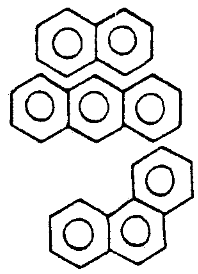 (SU=2),

anthracene (SU=3) and

phenanthrene (SU=3),

and the like.

The cycloaliphatic rings are substituted or un-substituted $C_5$ or $C_6$ hydrocarbon radicals. Substituted cycloaliphatic rings are analogous to substituted aromatic rings. Un-substituted rings include, by way of example, cyclopentane, cyclohexane and cyclohexene. Annulation of cycloaliphatic rings gives rise to

decalin 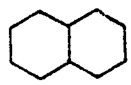 (SU=2)

[2.2.2]bicycloctane (SU=3)

norborane (SU=3)

adamantane ($C_{10}H_{16}$) 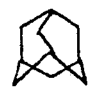 (SU=4)

and the like.

The term heterocyclic rings refers to substituted or un-substituted 5-6 membered heterocyclic radicals. Examples of 5-6 membered heterocyclic radicals are radicals of pyrrole, pyridine, furan, thiophene, imidazole, oxazole, thiazole, dibenzothiophene and carbazole.

Regarding the selection of heterocyclic structures, O and S heterocycles are generally suitable. In the case of N derivatives, however, care must be exercised such that the N is not strongly basic so that homopolymerization of the epoxide occurs. For example,

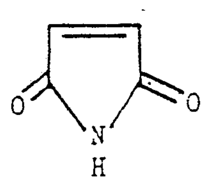

is suitable, but

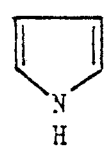

may not be suitable by itself (however the carbazole analogue is suitable since the N is not strongly basic there).

One group of diphenoxy components particularly useful herein are those mentioned in US-A 3,546,145. Specifically, those useful components are those phenoxy compounds of the formula

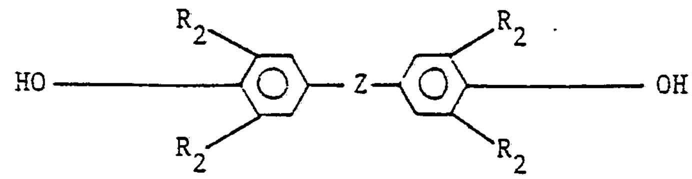

where each $R_2$ substituent is independently selected from H, Cl, Br or $C_1$-$C_5$ alkyl and Z is a substituent having flexible units (FU or FU') and stiff units (SU or SU') where Z represents a gem-bivalent radical having 1 or 2 aromatic hydrocarbon rings and a gem-bivalent non-aromatic ring selected from the group consisting of a ring of 5 carbon atoms, a ring of 6 carbon atoms one of which carbon atoms may bear an oxo oxygen atom, and a ring of 5 carbon atoms and one oxygen atom, said gem-bivalent non-aromatic ring being fused to said aromatic hydrocarbon rings. Particularly useful are those components where Z is

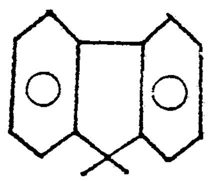

or

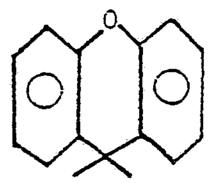

Specific examples include the following bisphenols:
9,9-bis(4-hydroxyphenyl)fluorene,
1,1-bis(4-hydroxyphenyl)-indane,
9,9-bis(4-hydroxyphenyl)xanthene,
10,10-bis(4-hydroxyphenyl)anthrone,
9,9-bis(4-hydroxyphenyl)phenanthrone.
Other useful bisphenols include phenolphthalene,
9,9-bis(4-hydroxyphenyl)-9,10-dihydroanthracene,
9,9-bis(4-hydroxyphenyl)-10,10-diphenyl-9,10-dihydroanthracene,
3,3-bis(4-hydroxyphenyl)-4,5-benzodihydrofuran, and the like.

Another group of diphenoxy components useful herein are the imides represented by the formula

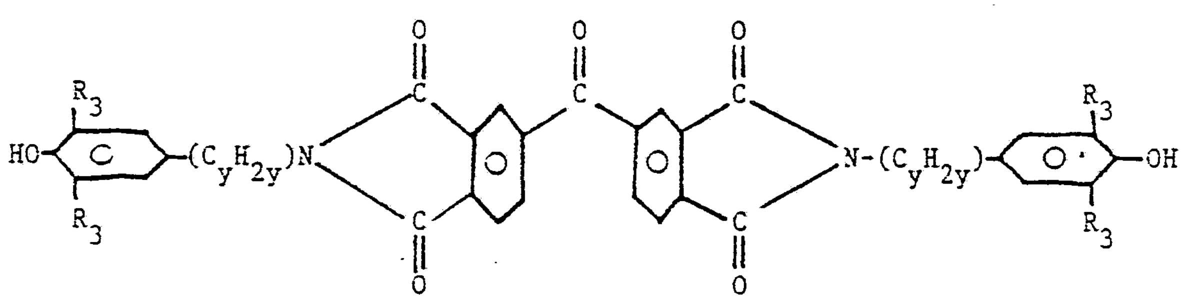

wherein each of $R_3$ is the same or different (lower) alkyl group of from one to four carbon atoms; and y has a value of from 0 to 3. Such diphenoxy compounds are disclosed in US-A 3,821,162.

Still another group of diphenoxy compounds are those based on phthalocyanine. Such compounds include $PcSi(R_5)_2$ in which $R_5$ = OH, or

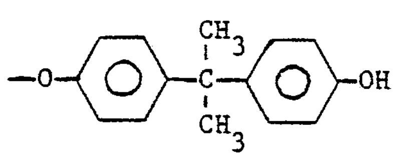

and Pc stands for a phthalocanine ring structure.

Still another group of diphenoxy compounds are those shown below. Additional aromatic, cycloaliphatic or heterocyclic rings may be annulated as desired:

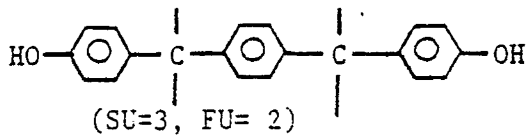

(SU=3, FU= 2)

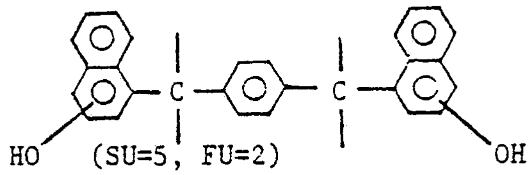

(SU=5, FU=2)

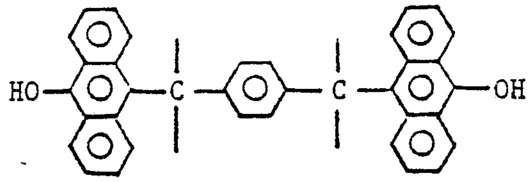

(SU=7, FU=2)

This particular group of diphenoxy compounds are distinguished from diphenoxy compounds such as BPA and the like, by the presence of 2 or more flexible units

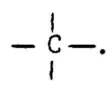

If desired, the diphenoxy compounds described above may be substituted in part (or even in whole in certain cases) with other diphenols, represented by the general formula

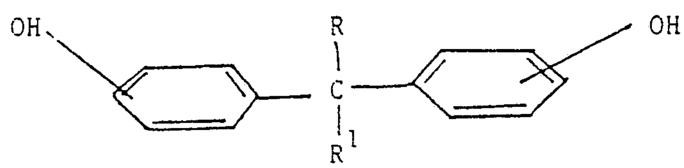

In which R and $R^1$ when taken collectively with the connector carbon C are selected from the group consisting of cyclohexyl and alkyl-substituted cyclohexyl, and when taken separately are from the group consisting of hydrogen, alkyl, cyclohexyl, phenyl, alkyl-substituted cyclohexyl, alkyl substituted phenyl, halogen substituted cyclohexyl and halogen substituted phenyl groups with the total number of carbon atoms in the group or groups attached to said connector carbon atom not exceeding eighteen and the number of carbon atoms in any of said alkyl substituent groups not exceeding six. The preferred phenols have the hydroxyl groups in the 4,4′ positions, but compounds with hydroxyls in the 2,2′, 3,3′, 2,4′ and other arrangements may also be used. R and $R^1$ suitable are methyl, ethyl, isobutyl, n-nonyl, n-heptadecyl and the like. Other dihydric phenols may also be employed, excepting those which have two hydroxyl groups in ortho positions on a single benzene ring.

B Diepoxide Component

The second reactant in the condensation process, the diepoxide, is a compound having two vicinal epoxide groups (oxirane rings) in terminal (or optionally non-terminal) positions in the molecule, usually in the form of an oxygen atom bound to two terminal carbons of an alkyl group, though the epoxide may also be on a ring, such as a cyclohexyl ring. Suitable diepoxides are terminal diepoxyalkanes, e.g., 1,2-epoxy-3,4-epoxybutane, 1,2-epoxy-5,6-epoxyhexane, 1,2-epoxy-7,8-epoxyoctane and the like. Others are terminal diepoxides containing ether linkages, such as bis(2,3-epoxypropyl)ether and bis(2,3-epoxy-2-methylpropyl)-ether; diglycidyl ethers of alpha, omega glycols such as the diglycidyl ethers of ethylene glycol, trimethylene glycol, and tetramethylene glycol; and diglycidyl ethers of dihydric phenols.

Diglycidyl ethers of the dihydric phenols referred to above are generally suitable for use in this invention. One may suitably use the diglycidyl ether of the same phenol which is employed as the other reactant. Thus, for example, bisphenol fluorenone is suitably condensed with diglycidyl ether of bisphenol fluorenone. Useful resins can also be prepared by condensing a dihydric phenol with the diglycidyl ether of a different dihydric phenol. For example, useful condensation products have been prepared according to this invention from the diglycidyl ether of BPA and the dihydric phenol prepared from phenol and fluorenone.

In preparing the products of this invention the epoxy reagent may be a pure diepoxide or a crude mixture containing a substantial proportion of diepoxide, e.g., 70% or more. It is important, however, that the crude reagent is free of monoepoxide and of monohydric alcohol or phenol. The polyepoxides used herein can have the structure

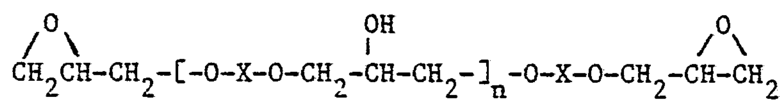

or

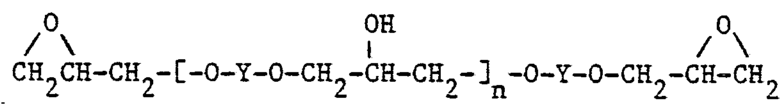

The number "n" has a value of from 0 to 6, preferably from 0 to 2, most preferably zero.

A particularly preferred diepoxide is the diglycidyl ether of BPA. Such diepoxides are available from Shell Chemical Company as EPON Resins 825 and 828. Shell EPON Resins 825 and 828 are diepoxides of BPA.

C. Selection of Stiff Units and Flexible Units for Stiff Segments and Flexible Segments

A key aspect of the present invention is the selection and location of the stiff units (SU and SU') and flexible units (FU and FU') for the stiff segments (X) and flexible segments (Y). As discussed above, the stiff segment (X) may be located in either the diphenoxy component or diepoxide component or in both components. For ease of synthesis it is preferred that the stiff segment be in either the diepoxide component or the diphenoxy component. The selection will depend upon the particular components to be employed. For example, since the diepoxide of BPA is readily available, it may be preferable to use such a resin in the synthesis. Since the diepoxide of BPA has one flexible unit and two stiff units, it has an $\frac{FU'}{SU'}$ of $\frac{2}{1}$ or 2 and will therefore be the flexible segment Y. Then one must use a diphenoxy component having sufficient number of stiff units (SU) and flexible units (FU) such that the ratio

$$\frac{\left(\frac{a}{a+b}\right) SU + \left(\frac{b}{a+b}\right) 2}{\left(\frac{a}{a+b}\right) FU + \left(\frac{b}{a+b}\right) 1}$$

is greater than 4. Where a/a + b is 0.5 then

$$\frac{0.5\ SU + 1}{0.5\ FU + .5} > 4$$

and therefore SU > 4FU + 2

For example, the bisphenol of fluorenone has five stiff units and zero flexible units. Accordingly, the reaction product of a 50:50 mixture of the bisphenol of fluorenone and the diglycidyl ether of BPA has a ratio of $\frac{SU}{FU}$ of $\frac{5}{0}$ and a ratio of $\frac{SU'}{FU'}$ of $\frac{2}{1}$

The average of

$$\frac{\left(\frac{a}{a+b}\right) SU + \left(\frac{b}{a+b}\right) SU'}{\left(\frac{a}{a+b}\right) FU + \left(\frac{b}{a+b}\right) FU'}$$

is $\frac{0.5 \times 5 + 0.5 \times 2}{0.5 \times 0 + 0.5 \times 1} = 7$, which is the average number of total stiff units divided by the average number of total flexible units.

More particulars on these ranges and selections are found in the discussion of the Structures of the Resulting Polymers.

## D. Catalyst and Reaction Conditions

The condensation reaction between the diphenoxy component and the diepoxide component requires the presence of a condensation catalyst, typically a basic condensation catalyst. The catalyst, may, for example, be added as a concentrated aqueous solution of sodium or potassium hydroxide or a sodium salt of a phenol. One may also use halides, carboxylates or other nucleophiles. It is sometimes desirable to use as catalyst a sodium salt of the same dihydric phenol which is used as a reactant. These salts are generally solids which are dissolved in the reaction mixture. It has been found that very satisfactory results are also obtained when using concentrated aqueous sodium hydroxide or benzyltrimethyl ammonium hydroxide. When the catalyst is added as an aqueous solution, a concentrated solution is used since it is not desirable to have more than a small amount of water present in the reaction mixture.

The concentration of catalyst present during the condensation reaction must be held to a low value; otherwise branched polymers of low impact value are produced. However, it has also been found that reaction rates increase proportionately with catalyst concentration. The useful range of catalyst concentration is from 0.0001 to 0.100 mole per mole of the contained bisphenol. For best results the concentration is preferably between 0.001 and 0.010 mole per mole. It may occur that some of the catalyst reacts with impurities in the reactants. This results in a reduction of the rate of reaction and can stop the reaction

prematurely. Adding a further amount of catalyst then permits the reaction to continue. It has been found that basic catalyst reacts with saponifiable chlorine if the latter is present in the diglycidyl ether reactant. It is therefore useful to add initially an extra amount of catalyst, sufficient to react with such chlorine, to prevent slowing down of the reaction.

It is preferred to keep the water content of the reaction mixture as low as possible, preferably below 0.5% by weight, more preferably below 0.2% by weight, and still more preferably below 0.12% by weight. In any event, the water content is to be maintained so as not to exceed about 1 percent by weight.

Careful control of the ratio of dihydric phenol and diglycidyl ether in the reaction mixture is of greatest importance in order to obtain a product having the desired characteristics. When technical grades of one or several reagents are employed, the correct ratio is maintained by determining the epoxy equivalence and the phenolic hydroxide equivalency of the reagents and carrying out the reaction with a mixture which contains not less than 0.90 phenolic hydroxide group per epoxide group and not more than 1.04 phenolic hydroxide group per vic epoxide groups

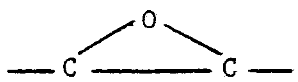

Best results are obtained with phenolic hydroxide/epoxy ratios in the range from 0.94 to 1.0. A slight excess of epoxy groups is preferred to a small excess of phenolic hydroxyl groups. When the catalyst employed is a basic salt of a dihydric phenol, then the phenol present in the catalyst is considered part of the phenolic reagent for purposes of calculating the proper ratio of reactants. Similarly, when the technical grade of the diepoxide contains some saponifiable chlorine, the chlorohydrin groups are considered the equivalent of epoxy groups since they are converted thereto during the condensation reaction in the presence of a basic catalyst. It is also desired to keep the saponifiable chlorine content low.

The reaction is typically carried out in solution in a solvent which meets the following criteria: (1) It is capable of maintaining reactants and reaction products in solution, at reaction temperatures, in the concentrations employed. These concentrations generally range between 20 and 60 percent by weight of the total reaction mixture. When the original concentration is high, it is generally necessary to add additional solvent during the course of the reaction to reduce the viscosity of the mixture and to maintain the product in solution. (2) It does not react significantly with epoxide groups or phenolic hydroxyl groups. Water and alcohols, for example, tend to interreact with the reactants and are therefore not suitable as solvents. (3) It is readily and completely removable from the final reaction mixture to permit recovery of a resin substantially completely free of solvent. Desired high impact resistance is a property which requires complete removal of solvent. In the production of resin for use in moulding, extrusion, and the like, solvent is removed from the reaction mixture. In the production of resin for surface coatings, the resin may remain associated with solvent until it is actually applied as a coating and the solvent is removed by evaporation under suitable conditions. (4) Its boiling point must be such that the reaction can be carried out at 75 °C to 150 °C at a practical pressure. The solvent may be a mixture of individual compounds.

Useful solvents which meet those criteria are, for example, certain ketones, halogenated hydrocarbons and ethers. Methyl ethyl ketone is a preferred solvent. Cyclohexanone, methyl isobutyl ketone and the other ketones may be used. Chloroform, 1,2-dichloroethane and other chlorinated hydrocarbons may be used, particularly in admixture with ketones. Ethers, such as dioxane, tetrahydrofuran, dimethoxyethane and lower alkyl (methyl or ethyl) ethers of ethylene glycol are suitable, alone or in admixture with ketones. Other solvents which meet the above criteria may be employed if desired, such as N-methyl pyrrolidone.

While in the examples which follow the synthesis was performed in solution, it is also possible (and desirable in some cases) to do the synthesis in the absence of solvent, i.e. as a melt. In such cases it may be desirable to use the diepoxide containing the stiff segment since the melting point of diepoxide component is usually much lower than the melting point of the corresponding diphenoxy component.

A necessary process step for obtaining solid resin of high impact resistance suitable for moulding or other forming is the complete removal of solvent from the resin mass.

## II. Resulting Polymers

### A. Structures

As mentioned above, a key aspect of the present invention is the selection and location of the stiff units (SU and SU') and the flexible units (FU and FU') for the stiff segments (X) and flexible segments (Y). Great latitude is provided for the selection and location of the particular components. For the most part the properties and performance of the resulting polymers depend primarily on the relative number of stiff units and flexible units (each such unit being assigned a value of one). However, there are certain important ratios and values that need to be followed.

Note that it is not necessary that the segments contain flexible units. For example, BPFL does not contain flexible units and is perfectly satisfactory.

The first important ratio is that $\frac{SU}{FU}$ must be equal to or greater than $\frac{SU'}{FU'}$, preferably greater than one. In other words, the stiff segment (X) must have an equal or higher ratio of $\frac{SU}{FU}$ than the flexible segment (Y) ratio of $\frac{SU'}{FU'}$. This is important because it helps determine the Tg/toughness balance.

Preferably $\frac{SU}{FU} > \frac{SU'}{FU'} + 0.5$.

In a preferred embodiment $\frac{SU'}{FU'}$ is between 1 and 4, preferably between 2 and 3. For example, where it is preferred to use the diglycidyl ether of BPA, the $\frac{SU'}{FU'}$ ratio is $\frac{2}{1}$ or 2, and when the diphenoxy compound used is the bisphenol of fluorenone, the ratio of $\frac{SU}{FU}$ is $\frac{5}{0}$ or infinity, and accordingly, $\frac{SU}{FU} > \frac{SU'}{FU'}$.

The second important ratio is the average number of total stiff units divided by the average number of total flexible units. For a preferred group of structures this ratio must satisfy the equation:

$$\frac{\left(\frac{a}{a+b}\right) SU + \left(\frac{b}{a+b}\right) SU'}{\left(\frac{a}{a+b}\right) FU + \left(\frac{b}{a+b}\right) FU'}$$

Preferably from 4 to 20, most preferably from 5 to 10. This ratio is important because it is an important factor in determining the Tg, or heat resistance of the polymer. In another preferred group of structures this ratio is less than one or equal to 4, preferably from 1.5 to less than 4, most preferably about 2.

The third important ratio is the relative amounts of stiff segments and flexible segments, i.e. $\frac{a}{a+b}$. In the broadest case the ratio of $\frac{a}{a+b}$ is more than zero and less than or equal to 1. The preferred ratios of $\frac{a}{a+b}$ are between 0.2 and 0.8, more preferably between 0.3 and 0.7, most preferably between 0.4 and 0.6. It is shown in the examples which follow that a most preferred ratio of $\frac{a}{a+b}$ is between 0.4 and 0.6 with

optimum overall properties (especially solvent resistance) occurring when the ratio of $\frac{a}{a+b}$ is equal to 0.5

B. Light Crosslinking

Another important aspect of the present invention relates to the light crosslinking of the thermoplastic polymer molecules to form the resulting polymer matrix. The concept and process for light crosslinking of such polymers is another novel and unobvious aspect of the present invention. In the broadest sense, light crosslinking refers to the crosslinking of between 1 and 50 out of each 100 repeat units to repeat units of other molecules, e.g. formulas I or II of said thermoplastic polymer. Preferably, the crosslinking density is between 2 and 20 out of 100, more preferably between 3 and 10 repeat units per 100 repeat units.

"Light crosslinking" is distinguished from the normal crosslinking or curing of epoxy resins where the crosslink density approaches 100 (stoichiometric) molecules or repeat units per 100 molecules or repeat units.

There are basically three different techniques that may be used to obtain lightly crosslinked matrices. One technique involves the use of a slightly greater number of diepoxide groups than phenolic groups (see earlier section on I.D. Catalyst and Reaction Conditions). When using this technique the repeat units will crosslink through the reaction of the secondary hydroxyl groups with the remaining epoxide groups. Once the thermoplastic polymer is prepared, it may be used alone or with a reinforcing fibre in an FRC-type (fibre reinforced composite) composition, wherein the polymer mass is heated to an elevated temperature (e.g. above 170 °C) and held at that temperature for the necessary time (typically about 2 to about 24 hours) to obtain crosslinking.

Another technique to obtain light crosslinking is to incorporate an appropriate amount of tri-or higher functional epoxide or tri-or higher functional phenolic or amine in the preparation of the thermoplastic polymer. The crosslinking agent, when added as a separate component, replaces a portion of the phenolic component or the epoxide component, as desired. For example, if 20% crosslinking agent is used, then 20% of the phenolic component is replaced on an equivalent basis.

Examples of suitable multifunctional epoxide polymers include Epon Resin 1031 and Epon Resin DPS-164. Epon Resin DPS-164 has the general formula

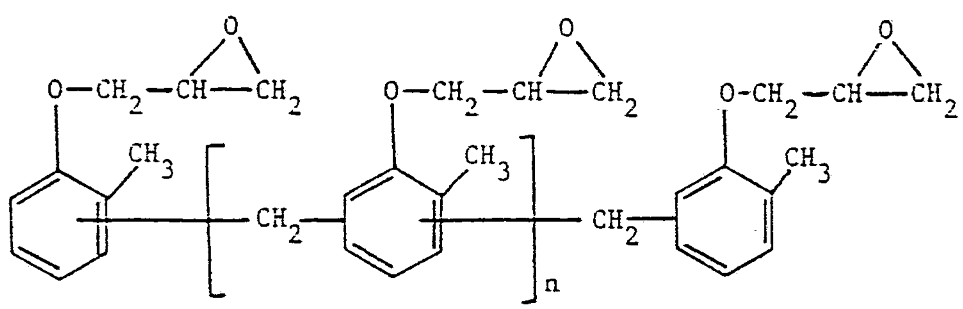

where n equals an average of 3.

Epon Resin 1031 has the structure

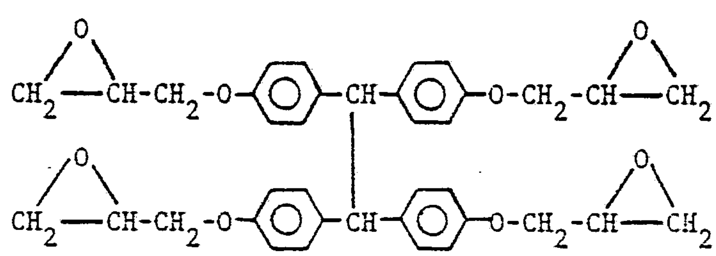

Other crosslinking agents include multifunctional amines such as EPON HPT™ Curing Agents 1061 and 1062, having the molecular structure:

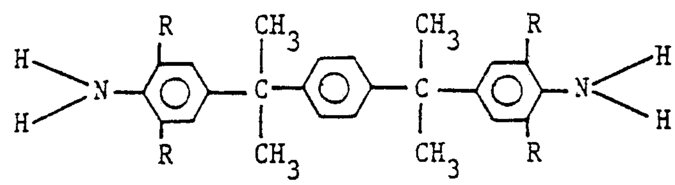

where R is H for CA 1061 and R is $CH_3$ for CA 1062.

Still other crosslinking agents include

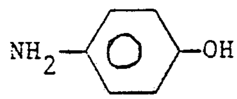 (PAP)

and

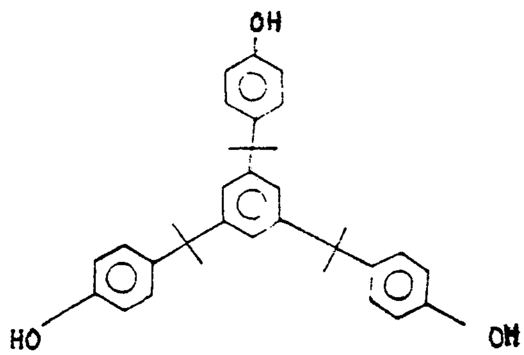

(TPA)

A third technique to obtain light crosslinking involves the addition of crosslinking agents, such as triepoxides, etc., to the resulting thermoplastic polymer. This technique is not preferred since it is more difficult to incorporate the crosslinking agent in the polymer after synthesis than before synthesis.

The amount of crosslinking agent chosen is selected to achieve the desired level of light crosslinking, as opposed to the normal crosslinking used for epoxy resins. Accordingly, when using a crosslinking agent such as EPON Resin 1031, the amount of equivalents used is 2 to 20%. Likewise, when the crosslinking agent is EPON HPT Curing Agent 1061, the amount of equivalents used is 5-50%.

C. Formulations and Composites

The composition optionally, but preferably for high-performance applications such as automotive and aerospace, contains a reinforcing substrate. Suitable reinforcing materials include, for example, glass fibers, carbon fibres, Kevlar, boron, calcium carbonate, talc, alumina, asbestos and the like. The preferred fibrous reinforcing material for high-performance applications is selected from the group consisting of glass fibres, carbon fibres, boron fibres and Kevlar fibres, with continuous carbon fibre being most preferred. The fibrous reinforcing material will be present in the composition in an amount effective to impart increased strength to the cured composition, generally from about 40 to about 95 weight percent, usually from about 60 to about 80 weight percent, based on the weight of the total composition.

The present composition can be applied to the fibrous reinforcing material from the melt or solution by methods known in the art. Among the various processes useful with the present invention include resin transfer moulding (RTM), pultrusion, filament winding and the use of prepregs. Such methods are known in the art.

One method of current preferred interest involves the use of prepregs. In that system, the polymer composition/curing agent - impregnated substrate, or "pre-preg", or a laminate prepared from a plurality of prepregs, is then cured. When the system is based on Epon® 825 resin and the bisphenol of fluorenone, the curing is typically accomplished at a temperature of about 150 to about 200 °C for about 1 to 16 hours under vacuum or under a presence of 1 to 20 bar to form the structural composite article.

D. Uses

The compositions of the present invention have particular application in the aerospace industry where the high performance obtainable with the present invention is required. In particular, RIM may be used to prepare large parts, such as helicopter blades. Prepregs may be used to prepare parts such as wings and the like. Filament winding may be used to prepare an entire fuselage, while pultrusion may be used to prepare parts having a constant cross section.

The invention composition can optionally include additives for control or modification of various properties of the composition in its cured or uncured state, including cure rate accelerators or retardants, tackifiers and the like.

To illustrate the present invention, the following illustrative embodiments and comparative examples are given. It is to be understood, however, that the embodiments and examples are given for the purpose of illustration only and the invention is not to be regarded as limited to any of the specific materials or conditions used in the specific embodiments.

As used in the following examples, Epoxy Resin A is a liquid glycidyl polyether of 2,2-bis(4-hydroxyphenyl)propane having an epoxide equivalent weight of 170-174 and an average molecular weight of about 345.

Epoxy Resin B is a liquid glycidyl polyether of 2,2-bis(4-hydroxyphenyl)propane having an epoxide equivalent weight of 180-195 and an average molecular weight of about 380.

Epoxy Resin C is a solid glycidyl polyether of 2,2-bis(4-hydroxyphenyl)propane having an epoxide equivalent weight of 1650-2100 and an average molecular weight of about 2900.

The compositions were tested according to the following test procedures:

Flexural properties of neat resins were evaluated according to ASTM D790 method using 1/8 in. thick specimens. Specimens were tested both in Dry (at Room Temperature and ~75% R.H.) and Hot/Wet (after immersion in boiling water for 48 hours, test at 95 °C, 5 min. equilibration time) conditions.

Fracture toughness, $K_q$, was measured using mini-compact tension specimens (see W.B. Jones, et al Am. Chem. Soc., Div. Polym. Chem., Polym. Prepr., 22, 1981). All specimens were slotted to a Chevron shape and then precracked with a razor blade.

Tensile properties were measured according to ASTM D638 method.

Swelling in solvents was evaluated by measuring weight gain per unit of initial weight after immersion in solvent for a specified time at room temperature.

Illustrative Embodiment I

In this example, a thermoplastic polymer composition according to the present invention was prepared having a phthalocyanine structure as the stiff segment. Initially 1,3-diiminoisoindoline was prepared from 0-phthalonitrile by the method of Marks (see J.J. Marks, C.W. Dirk, T. Inabe and J.K. Schach, Jr., J. Am. Chem. Sci., 105 (6), 1539(1983) to give a green solid with a melting point (m.p.) of 193-194 °C in 50% yield.

A $SiCl_2$.phthalocyanine ($PcSiCl_2$) structure was then prepared from 1,3-diimino-isoindoline by the method of Marks. The purple solid was washed with chloroform, methanol, acetone and hexane to give product in 86% yield. For analysis the material was dried at 110 °C (1 mm) for 2 hours.

Ten grams of the above dichloride and 100 g of BPA, 6.7 ml of pyridine and 400 ml of o-dichlorobenzene were mixed under anhydrous conditions and stirred under nitrogen at 140 °C for 5 hours. The mixture was cooled, diluted with 400 ml of anhydrous THF and filtered. The filtrate was evaporated in-vacuo (water bath = 60-70 °C) to dryness. The residue was stirred with 1.3 l of 50% aqueous acetone to remove excess BPA, filtered, and washed with 2-100 ml portions of acetone to give 14.6 g (89%) of blue solid.

HPLC analysis (uncalibrated) showed the material to be about 92% pure $PcSi(BPA)_2$.

In the next step, varying amounts of PcSi(BPA)$_2$ were employed with Epoxy Resin A to prepare thermoplastic compositions according to the present invention. Recrystallized Epoxy Resin A, 8.824 mmol, dry N-methyl-2-pyrrolidone (NMP), 8 ml, NaBPA, 0.176 mmol, and 8.824 mmol of a mixture of BPA and PcSi(BPA)$_2$ were mixed under anhydrous conditions, and heated under $N_2$ at 110 °C for 5 hours. The mixture was cooled, evaporated at room temperature and $10^{-6}$ m pressure to dryness. The residue was extracted with acetone to constant $T_g$.

The results are presented below in Table 1:

TABLE 1

| mmol | PcSi(BPA)$_2$ Mole Fraction | $\bar{M}_n$ | $T_g$ (°C) | Solubility (%w, r.t) DMK | THF | NMP |
|---|---|---|---|---|---|---|
| 0 | 0.00 | 8,000 | 100 | >40 | >40 | >40 |
| 1.87 | 0.09 | 17,000 | 123 | 0.5 | 19 | >20 |
| 2.80 | 0.13 | -- | 136 | 0.1 | 10 | >20 |
| 4.67 | 0.18 | 10,000 | 151 | 0.05 | 4 | >20 |

As shown above, the $T_g$ increases and the solubility decreases rapidly with phthalocyanine content. At a phthalocyanine mole fraction of 0.5, the $T_g$ would be expected to be about 280 °C.

Illustrative Embodiment II

Bisphenol Ib was prepared in 98% yield by refluxing benzophenone tetracarboxylic dianhydride, E, and para-aminophenol, F, in acetic acid.

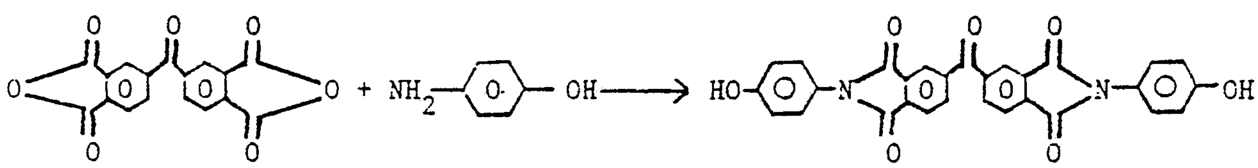

E(BTDA) F Ib

The above products also include the

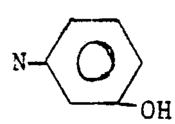

isomer

Specifically, BTDA (E), 32.2 g, and 21.8 g of p-aminophenol were stirred in 300 ml of acetic acid at room temperature for 7 hours and then refluxed overnight. The mixture was cooled, filtered, and the solid washed with 800 ml of water. The material was dried at 180 °C for 6 hours to give 49.5 g (98% yield) of yellow solid, m.p. (DSC) ~394 °C.

For analysis, the material was recrystallized from NMP-toluene, m.p. (DSC) 424 °C. The IR spectrum of this material showed the presence of imide and phenolic groups and the absence of anhydride groups.

Next, Epoxy Resin A, 8.824 mmol, 8.824 mmol of bisphenol Ib, 0.176 mmol Na BPA, and 30 ml of NMP were mixed and stirred under $N_2$ at 120 °C for 24 hours. The reaction mixture was cooled and precipitated by pouring into ice water in a Waring blender to give 7.3 g (97%) of yellow solid after drying. The material had a $T_g$ of 173 °C which increased to 175 °C after washing with NMP. The $M_n$ was 10,800 (OSMOMETRY). Solubility in THF, $CH_2Cl_2$ and Skydrol was nil, 1.5%w in NMP. The material exhibits a crystalline m.p. ~320 °C which does not shift from heat up to cool down. TGA shows the material is generally stable up to 375 °C, but exhibits very slow decomposition at 340 °C with consequent void formation during compression moulding at that temperature. The material can be extruded, but void formation is extensive.

A similar run was made using 90 %w Epoxy Resin A and 10 %w Epoxy Resin C (EQ.WT = 1676) to give 7.7 g of polymer, $T_g$ (DSC) = 157 °C, $T_m$ (DSC) = 320 °C. Solubility in NMP = 7.8 %w.

Illustrative Embodiment III

Illustrative Embodiment III compares various systems according to the invention with other competing systems. System 1 (outside the invention) is based on a compilation of data for the diaminodiphenyl sulphone curing of the tetraglycidyl ether of methylene dianiline.

Systems 2, 3 and 4 are based on the copolymer from Epoxy Resin A and bisphenol-fluorenone (BPFL). To prepare the composition designated System 2, recrystallized Epoxy A, 3.000 g, BPFL, 3.0227 g, and monosodium BPA, 0.0441 g, were mixed with 16 ml of NMP and heated at 120 °C under $N_2$ for 5 hours. The cooled solution was poured into excess ice water, filtered, extracted with acetone and toluene, and dried to give 5.7 g (94% yield) of white solid, $T_g$ = 162 °C, mol.wt. (OSMOMETRY) = 24,600, $[\eta]_{35\ °C,NMP}$ = 0.56. Alternatively, System 2 may be prepared by mixing BPFL with Epoxy A at a mole ratio (P/E) of 1.04, adding catalyst and heating to 180 °C, degassing at 1 Torr, and curing for 24 hours at 180 °C.

System 3 involved melt polymerization of BPFL and Epoxy A at a P/E ratio of 0.96 with 0.18 %m (basis Epoxy A) NaBPA catalyst. Reaction conditions were typically 190 °C for 24 hours.

System 4 involved melt polymerization of BPFL and Epoxy A under conditions identical to system 3 except that part of Epoxy A was replaced by an equivalent amount of Hycar® CTBN such that the final product contained 10 %wt Hycar CTBN (a butadiene rubber produced by B.F. Goodrich).

System 5 relates to a thermoplastic poly-(etheretherketone) matrix.

The results are presented below in Table 2. As shown in Table 2 by comparing System 2 with System 3, lightly crosslinking the epoxy/BPFL matrix significantly improves toughness ($K_q$) and tensile strength along with chemical resistance.

TABLE 2

MATRIX MECHANICAL PROPERTIES

| System No. | System | $T_g$ (°C) | Sol. %w, NMP | Process[a)] TP | Process[a)] TS | $K_q$ (psi. in.$^{0.5}$) | E (ksi) | $\varepsilon$[b)] (%) | $\sigma$[b)] (ksi) |
|---|---|---|---|---|---|---|---|---|---|
| 1 | TGEMDA/DDS[c)] | 210 | 0 | - | + | ∿ 500 | 560 | ∿ 2 | 16 |
| 2 | Epoxy Resin A/BPFL[e)] | 162 | 15 | + | + | 1500 | 400 | ∿ 2 | 8 |
| 3 | a) Crosslinked | 175 | 0 | + | + | 2500 | 400 | > 10 | 20 |
| 4 | b) 10 %w Rubber[d)] | 145 | 1 | + | + | 3200 | 350 | > 10 | 16 |
| 5 | PEEK | 145 | 0.1 | + | - | ∿ 4000 | 500 | > 10 | 15 |

a) TP = Thermoplastic, TS = Thermoset Processability; + means acceptable, - means unacceptable.

b) Flexural measurements.

c) Compilation data, approximate only. TGEMDA = TetraGlycidyl Ether of Methylene DiAniline, DDS = DiaminoDiphenyl Sulphone.

d) HYCAR CTBN.

e) Melt polymerization.

Illustrative Embodiment IV

The important factors affecting the development of new matrix resins for advanced composites are: toughness, moisture resistance, elevated temperature performance and improved processability. The importance of these factors invariably is the result of a particular deficiency in current resins relative to specific applications. Traditional methods of toughening epoxy resins by flexibilization or by introduction of rubber invariably result in the reduction of hot-wet properties of the resin. Thermoplastic matrices can have high toughness but usually suffer from chemical resistance or processability. An alternative approach is the development of "crosslinked thermoplastic" on the basis of epoxy chemistry, as disclosed and claimed herein.

This approach has resulted in the development of a new copolymer with a controllable degree of crosslinking such that it could cover the thermoplastic-thermoset range, which should allow the optimization of the system. The chemistry for the preparation of these materials is shown in Illustrative Embodiment III. The copolymer of Epoxy Resin A and bisphenol-fluorenone (BPFL) exhibited a good balance between fracture toughness and $T_g$ values. In the present example, other important characteristics (e.g. water absorption and hot-wet properties) have been studied.

Water absorption was measured by the specimen's weight gain as a function of time. The polymer was immersed in boiling water. As was shown from the shape of the curve of absorption, saturation takes place at approximately 300 hours. The weight gain at saturation is equal to 1.45%, which is typical for high temperature performance resins rather than for flexibilized epoxy resins.

The hot-wet properties of the Epoxy Resin A/BPFL copolymer were studied after its immersion for 40 hours in boiling water. Flexural properties were measured at room temperature, 95 °C and 136 °C both for dry and wet materials (see Table 3). All specimens exhibit yielding at approximately 5-6% of deformation. Specimens did not break at up to 10% strain in bending conditions with the exception of a few specimens which had large voids near the outer surface under the loading nose.

As shown in Table 3, the reduction in moduli with increasing temperature from 21 to 95 °C is minor and is practically the same for dry and wet materials indicating that in this temperature range the change in modulus can be considered primarily a function of temperature alone. At higher temperature, 136 °C, the effect of moisture presence is more pronounced since the decrease in modulus for wet material is significantly higher than for dry material. A similar interpretation can be made with respect to the change in yielding stress as a function of temperature and moisture.

On the basis of these observations the following conclusions can be drawn. The water sorption at saturation of EPON/BPFL copolymers is relatively low. The copolymer retains good mechanical properties under hot-wet conditions up to 95 °C and therefore can be considered a good candidate for aerospace applications.

TABLE 3

Flexural Properties of Epoxy Resin A/BPFL Copolymer

| | E, ksi | | $\sigma_y$, ksi | | εmax, % | |
|---|---|---|---|---|---|---|
| Temp., °C | D | W | D | W | D | W |
| 21 | 380 | 380 | 18.7 | 18.0 | > 10 | > 10 |
| 95 | 350 | 365 | 15.5 | 13.2 | > 10 | > 10 |
| 136 | 285 | 170 | 8.7 | 3.7 | > 10 | > 10 |

D - Dry

W. - Wet (After Immersion for 40 hr in Boiling Water)

Illustrative Embodiment V

Illustrative Embodiment V deals with the effect of small changes in crosslink density on polymer performance. The components employed were BPA, BPFL and Epoxy Resin A. The phenol to epoxy resin ratio was varied in order to vary the crosslink density from 0% (1:1 ratio of phenol to epoxy) to about 10% )0.9:1 ratio of phenol to epoxy). The syntheses of these polymers were similar to those prepared in Illustrative Embodiment III except as shown in Table 4.

The preparation conditions and test results are presented below in Tables 4 and 5. As may be seen from Table 5, there is the expected increase in glass transition temperature ($T_g$) from 166-172 °C, but the mechanical properties are roughly constant with the fracture toughness at a high value of 2330 ± 90 psi.in.$^{0.5}$. Closer examination of the fracture toughness data indicates an optimum value in the P/E range of 0.96-0.98. Similar data clearly shows reduced values at higher P/E ratios.

TABLE 4

| Experiment** | P/E | Catalyst %m* | Temp. (°C) | Time (hrs.) |
|---|---|---|---|---|
| 78 | 1.00 | 0.16 | 190 | 24 |
| 82 | 0.98 | 0.18 | 190 | 24 |
| 75 | 0.96 | 0.16 | 180 | 24 |
| 83 | 0.94 | 0.18 | 190 | 24 |
| 80 | 0.90 | 0.24 | 190 | 24 |

* Moles NaBPA/moles epoxide.

** 1/8'' castings in glass mould by melt polymerization at prescribed conditions.

TABLE 5

MECHANICAL PROPERTIES OF EPOXY RESIN A/BPFL COPOLYMER

| Experiment | Phenol/epoxy mol ratio | Crosslink[5] Density % | BPFL/BPA[4] mol ratio | $T_g$[1] (°C) | E[2] (KSI) | σy[2] (KSI) | εy[2] (%) | $K_q$[3] $psi.in.^{0.5}$ |
|---|---|---|---|---|---|---|---|---|
| 78 | 1.00 | 0 | 0.90 | 166 | 360 | 11.3 | 6.8 | 2300 |
| 82 | 0.98 | 2 | 0.90 | 168 | 390 | 11.2 | 6.5 | 2450 |
| 75 | 0.96 | 4 | 0.90 | 169 | 360 | 11.0 | 6.4 | 2350 |
| 83 | 0.94 | 6 | 0.90 | 172 | 380 | 11.2 | 7.1 | 2200 |
| 80 | 0.90 | 10 | 0.90 | 172 | 385 | 11.2 | 6.9 | -- |

1) DSC measurement

2) Tensile measurement

3) Mini compact tension

4) Ratio of BPFL to BPA moieties irrespective of source

5) % Branched epoxy groups/total epoxy groups

Illustrative Embodiment VI

Illustrative Embodiment VI deals with the preparation of polymers from 9,9-bis(4-hydroxyphenyl)-10-anthrone (BPAQ) and Epoxy Resin A. The BPAQ was prepared by reacting anthraquinone (AQ) with a 13 molar excess of phenol in the presence of 1.05 molar trifluoromethanesulphonic acid (triflic acid) for 60-90 minutes at 100 °C. BPAQ was recovered in 40-45% yield by neutralizing the reaction mass with 20 %wt aqueous NaOH and filtering the precipitate (which was 85-95% BPAQ). Purification involved recrystallization from boiling dimethylsulphoxide and then extraction of residual AQ with boiling acetic acid. The 99.5% pure BPAQ melts at 319-321 °C and has been identified by $C^{13}$ NMR analysis.

The diglycidyl ether of BPAQ (DGBPAQ) was prepared from BPAQ in a standard prep procedure. To prepare the polymers of the present invention, BPA (0.96 mole) and DGBPAQ (1.00 mole) were mixed with NaBPA (0.0012 mole), melted at 180 °C ($N_2$), poured into a glass mould and cured at 180 °C for 1 hour and 190 °C for 23 hours, $T_g$ = 175 °C (DSC).

Flexural and Fracture Toughness Data for BPA/BPAQ Polymer Matrix E = 400 KSI
$\sigma$Yield = 18.5 KSI
$K_q$ = 2400 psi.in.$^{0.5}$
$\epsilon$ > 8% (no break)
In a similar manner BPFL was reacted with DGBPAQ and NaBPFL catalyst at 210 °C for 24 hours, resulting in a polymer having a $T_g$ = 225 °C.

Illustrative Embodiment VII

Illustrative Embodiment VII deals with the preparation of Bisphenol-Fluorenone (BPFL) - Epoxy Resin A copolymer/graphite fabric composite. BPFL and Epoxy Resin A were mixed at 180 °C and then cooled to room temperature under conditions similar to that in Illustrative Embodiment III. At room temperature this product is in solid form and it has practically unlimited shelf life. The composite was prepared by a melt fusion method using a vacuum bag moulding technique. The BPFL-Epoxy Resin A compound was powderized and placed between layers of graphite fabric (Magnamite Graphite Fabric Style AS 370-8H). It was covered with release fabric, bleeder and vacuum bag. Curing conditions were the following: vacuum: 30 in Hg, top pressure: 90 psi, temperature: 180 °C for 1 hour and 190 °C for 16 hours. This resulted in a practically void free composite containing 62% fibre by volume.

The following mechanical properties were measured: Flexural (ASTM D790=, Short Beam Shear (SBS) strength, Mode I Interlaminar Fracture Toughness (see P.E. Keary and L.B. Ilecevicz, J. Comp. Mater., Vol. 19, March 1985, pp. 154-177). This data along with the data from the literature for conventional epoxy matrix/graphite fabric composites are presented in Table 6. As can be seen, the BPFL-Epoxy Resin A matrix composite has flexural and short beam shear properties comparable to conventional epoxy matrix composites, while its delamination fracture toughness is an order of magnitude higher than for conventional epoxy matrix composites. That high level of fracture toughness in combination with good traditional properties and high glass transition temperature (172 °C) allows one to consider the BPFL-Epoxy Resin A copolymer as an excellent matrix material for high performance composite materials.

TABLE 6

| | BPFL-Epon Resin A/ Graphite Composite | Conventional Epoxy/ Graphite composite |
|---|---|---|
| Flexural Strength (KSI) | 134 | 130[a)] |
| Flexural Modulus (KSI) | 9100 | 9500[a)] |
| SBS Strength (KSI) | 9.7 | 9.0[a)] |
| Delamination Fracture Toughness ($in.lbs/in^2$) | 11.0 | 1.3[b)] |

a) Hercules Product Data Sheet.

b) J.T. Hartness, SAMPE, January 1983.

Illustrative Embodiment VIII

In Illustrative Embodiment VIII, various polymers were prepared according to the present invention in syntheses similar to those used in Illustrative Embodiment III. The various formulations and test results for cured compositions are shown below in Table 7.

TABLE 7

| Phenol | Epoxy | P/E | Cat(%)[1)] | Cure[2)] | Gel(%) | $T_g$ (°C)[3)] | %wt GAIN[4)] MEK | $CH_2Cl_2$ | $H_2O$ | FLEX DATA E(KSI) | (KSI) | ε(%) | $K_q$ (psi.in.$^{0.5}$) |
|---|---|---|---|---|---|---|---|---|---|---|---|---|---|
| BP | DGBPFL | 0.96 | 75 | B | 100 | 194 | 7 | 42 | | 340 | 16.5 | > 8 | 1600 |
| BPA | DGBPFL | 0.96 | 75 | A | 86 | 175 | | | | 400 | | | 1700 |
| BPA | DGBPAQ | 0.96 | 50 | A | 80 | 175 | | 112 | | 400 | 18.5 | > 8 | 2400 |
| BPC | DGBPFL | 0.96 | 75 | B | 94 | 185 | 23 | 75 | 2.2 | 440 | 16.7 | 4 | 1500 |
| HFBPA | DGBPFL | 0.96 | 75 | A | 96 | 188 | | 200 | | 400 | 20.5 | 7 | 1400 |
| BPFL | DGBPA | 0.96 | 75 | A | 98 | 175 | 25 | 113 | 1.8 | 390 | 20.5 | > 8 | 2500 |
| BPFL | DGBPFL | 0.96 | 75 | B | 32 | 222 | 39 | 303 | 1.5 | | | | 700 |
| BPFL | DGBPAQ | 0.96 | 30 | B | 46 | 222 | | | | | | | |

STRUCTURE LEGEND

| SYMBOL | CHEMICAL NAME |
|---|---|
| BP | 4,4'-Dihydroxybiphenyl |
| BPA | 2,2-Bis(4-hydroxyphenyl)propane |
| BPC | 4,4'-Dihydroxybenzophenone |
| HFBPA | 1,1,1,3,3,3-Hexafluoro-2,2-bis(4-hydroxyphenyl)propane |
| BPFL | 9,9-Bis(4-hydroxyphenyl)fluorene |
| BPAQ | 9,9-Bis(4-hydroxyphenyl)-10-anthrone |
| DGBPA | 2,2-Bis[4-(2,3-epoxypropoxy)phenyl]propane |
| DGBPFL | 9,9-Bis[4-(2,3-epoxypropoxy)phenyl]fluorene |
| DGBP | 4,4'-Bis(2,3-epoxypropoxy)bisphenyl |
| DGBPAQ | 9,9-Bis[4-(2,3-epoxypropoxy)phenyl]-10-anthrone |

1) 100% = 0.12 %m, basis epoxide.

2) Cure A is 190 °C for 24 hours, Cure B is 210 °C for 24 hours.

3) DSC.

4) 24 hours at room temperature for MEK, $CH_2Cl_2$, Equilibrium for $H_2O$.

5) Mini-compact tension.

TABLE 8

| Run | Phenol Type, mole % | Epoxy Type | Crosslinking Agent Type | Crosslinking Agent Percent | P/E Molar ratio | Catalyst[1] Percent Theoretical | SU/FU | SU'/FU' | average of stiff units divided by average of flexible units |
|---|---|---|---|---|---|---|---|---|---|
| 1 | BPA, 100 | DGBPA | -- | -- | 0.96 | 75 | 2 | 2 | 2 |
| 2 | BPA, 80 | DGBPA | -- | -- | 0.96 | 75 | 2/1 | 2 | 2.57 |
| | BPFL, 20 | | | | | | 5/0 | | |
| 3 | BPA, 40 | DGBPA | -- | -- | 0.96 | 75 | 2/1 | 2 | 4.17 |
| | BPFL, 60 | | | | | | 5/0 | | |
| 5 | BP, 100 | DGBPA | -- | -- | 0.96 | 30 | 2/0 | 2 | 4 |
| 6 | BPA, 100 | DGBPA | EOCN | 5 | " | 100 | 2 | 2 | 2 |
| 7 | " | " | " | 20 | " | " | 2 | 2 | 2 |
| 8 | " | " | " | 30 | " | " | 2 | 2 | 2 |
| 9 | " | " | " | 50 | " | " | 2 | 2 | 2 |

1) 100% = 0.24 %m basis epoxy

TABLE 9

Properties

| Run | Tg °C | $K_q$ PSI | %wt gain (RT,Eq) MEK | $CH_2Cl_2$ | $H_2O$ | Flex Data (KSI) E,dry | E,h/w | Stress | Strain ε | Gel % |
|---|---|---|---|---|---|---|---|---|---|---|
| 1 | 115 | 3600 | 65 | 123 | 2.5 | | | | | 100 |
| 2 | 128 | 3000 | | | | 396 | | 14.6 | > 7 | |
| 3 | 153 | 2000 | | | | 375 | | 17.5 | 9 | |
| 5 | 128 | 4300 | | | | 280 | | 12 | > 8 | |
| 6 | 121 | 2400 | | | | 370 | | 14.6 | 8 | |
| 7 | 130 | 1900 | | | | 390 | | 17.6 | 10 | |
| 8 | 143 | 1900 | | | | 410 | | 19 | 7 | |
| 9 | 169 | 600 | | | | 426 | | 20.5 | 8 | |

Illustrative Embodiment IX

Polymerization of BPA and EPON 825 Resin

All of the materials used were recrystallized and thoroughly dried in a vacuum oven at 1 mm Hg vacuum before they were used.

In this example no separate crosslinking agent was used. Instead the phenol/epoxy ratio was 0.96. The components used were BPA and Epoxy Resin A.

The polymer of the present invention was made by melting the BPA and Epoxy Resin A together in the presence of a Na BPA catalyst at 180 °C in a vacuum erlenmeyer flask, degassing the melt at 1 mm Hg until bubbling ceased, pouring the molten pre-polymer into a mould (made of two glass plates treated with a releasing agent preheated in a forced draft oven at 180 °C) and curing the polymer for 24 hours. At the end of the cure cycle, the polymer-in-mould was taken out of the oven and allowed to cool below its Tg and then the plates were popped loose.

The following properties were obtained:

$T_g$ = 115 °C

$K_q$ = 3600 psi.in.$^{0.5}$

Gel = 100%

Illustrative Embodiment X

Various other polymers were prepared according to the invention. The various materials used were:

| Symbol | Chemical Name |
|---|---|
| BP | 4,4'-dihydroxybiphenyl |
| BPA | 2,2-bis(4-hydroxyphenyl)propane |
| DGBPA | 2,2-bis[4-(2,3-epoxypropoxy)phenyl]propane |
| BPFL | 9,9-bis(4-hydroxyphenyl)fluorene |
| EOCN | EPON Resin DPS-164 |

These polymers were prepared in a manner similar to that used in Illustrative Embodiment IX. The results are shown in Tables 8 and 9. Run 1 is the same as that described in Illustrative Embodiment IX.

## Claims

1. A composition comprising linear molecules having the repeating structures:

$$-\!\!\left( O\text{-}X\text{-}OCH_2\overset{\displaystyle OH}{\overset{|}{C}}HCH_2 \right)\!\!- \quad \text{and} \qquad \text{I}$$

$$-\!\!\left( O\text{-}Y\text{-}OCH_2\overset{\displaystyle OH}{\overset{|}{C}}HCH_2 \right)\!\!- \quad \text{where} \qquad \text{II}$$

a) "X" represents a stiff segment comprising stiff units (SU) and optional flexible units (FU), which stiff units and flexible units are interconnected;

b) "Y" represents a flexible segment comprising stiff units (SU') and optional flexible units (FU') which stiff units and flexible units are interconnected;

c) said stiff units, SU and SU', are independently selected from the group consisting of substituted and non-substituted aromatic rings, cycloaliphatic rings and non-interfering heterocyclic rings;

d) said flexible units, FU and FU', are independently selected from the group consisting of

$$-\overset{|}{\underset{|}{C}}-,\quad \overset{|}{\underset{/\ \backslash}{N}},\quad \underset{/\ \backslash}{O},\quad -\overset{|}{\underset{|}{Si}}-,\quad \overset{|}{\underset{/\ \backslash}{B}}\quad \text{and}\quad \underset{/\ \backslash}{S};$$

e) the number of stiff segments in said molecules is "a", the number of flexible segments in said molecules is "b", and the ratio $\frac{a}{a+b}$ is less than or equal to one; and

f) the ratio of the number of stiff units to flexible units in said stiff segment (SU/FU) is equal to or greater than the ratio of the number of stiff units to flexible units in said flexible segment (SU'/FU').

2. A composition as claimed in claim 1, in which the number of stiff units (SU, SU') and flexible units (FU, FU') is selected such that

$$\frac{a}{a+b}.SU + \frac{b}{a+b}.SU$$

divided by

$$\frac{a}{a+b}.FU + \frac{b}{a+b}.FU$$

is greater than four.

3. A composition as claimed in claim 1, in which the number of stiff units (SU, SU') and flexible units (FU, FU') is selected such that

$$\frac{a}{a+b}.SU + \frac{b}{a+b}.SU'$$

divided by the average number of total flexible units

$$\frac{a}{a+b}.FU + \frac{b}{a+b}.FU'$$

is less than or equal to four.

4. The composition of any one of claims 1 to 3, wherein the average number of total stiff units (SU + SU') divided by the average number of total flexible units (FU + FU') is between 5 and 10.

5. The composition of claim 3, wherein the average number of total stiff units (SU + SU') divided by the average number of total flexible units (FU + FU') is between 1.5 and 4.

6. The composition according to any one of claims 1 to 5, wherein the stiff segment X has the structure -Ar-Z-Ar-where Ar is substituted or non-substituted benzene rings, optionally annulated with one or more additional benzene rings, and wherein Z represents a gem-bivalent radical having 1 to 2 aromatic hydrocarbon rings and a gem-bivalent non-aromatic ring selected from the group consisting of a ring of 5 carbon atoms, a ring of 6 carbon atoms one of which carbon atoms may bear an oxo oxygen atom, and a ring of 5 carbon atoms and one oxygen atom, said gem-bivalent non-aromatic ring being fused to said aromatic hydrocarbon rings.

7. The composition of any one of the claims 1 to 6, which additionally contains an effective amount of crosslinking agent such that upon curing between 1 and 20 repeating structures from different molecules per 100 of said repeating structures are crosslinked.

8. A composition resulting from the curing of the composition of claim 7.

9. The cured composition of claim 8 having a glass transition temperature of at least about 150 °C, a flex modulus of at least 350 KSI and a fracture toughness of at least 1000 psi.in.$^{0.5}$

10. A composition as claimed in claim 7, wherein -Z-is

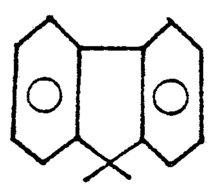

or

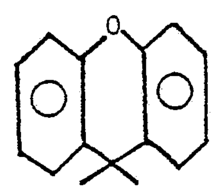

11. A composition as claimed in claim 1, in which X is

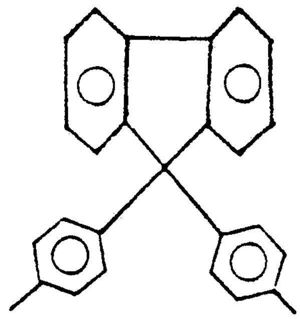

or

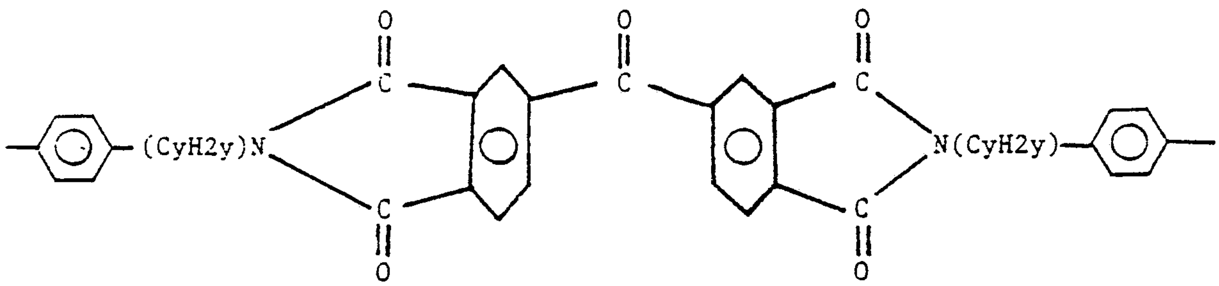

and "y" has a value of from 0 to 3.